# EUROPEAN PATENT SPECIFICATION

(11) **EP 1 600 111 B1**
(45) Date of publication and mention of the grant of the patent: **03.01.2007**
(21) Application number: 05291081.7
(22) Date of filing: 19.05.2005
(51) Int. Cl.: A61B 17/70

(54) **Device for connecting the rod of a spinal osteosynthesis device to a vertebra, and osteosynthesis system comprising this device**
Vorrichtung zur Verbindung des Stabes einer Osteosynthesevorrichtung für die Wirbelsäule an einem Wirbelknochen und Osteosynthesesystem mit dieser Vorrichtung
Dispositif de connexion de la tige d'un dispositif d'ostéosynthèse rachidienne à une vertèbre et système d'ostéosynthèse comprenant le dispositif

(30) Priority: 26.05.2004 FR 0405690
(43) Date of publication of application: 30.11.2005
(73) Proprietor: Warsaw Orthopedic, Inc., Warsaw, IN 46581 (US)
(72) Inventor: Gournay, José, 77230 Dammartin en Goele (FR); Demay, Gaspard, 75014 Paris (FR); Hovorka, Istvan, 06200 Nice (FR)
(74) Representative: Neyret, Daniel Jean Marie

(56) References cited:
- EP-A- 0 846 444
- WO-A-01/58369
- FR-A- 2 767 263
- FR-A- 2 816 196
- NL-C- 1 011 260
- US-A- 5 498 263

## Description

The invention concerns the field of spinal osteosynthesis devices.

Spinal osteosynthesis devices for correcting deformations of the spine often comprise at least one rod extending along a portion of the vertebral column of the patient. It is connected to the vertebrae by devices which can take various forms. In particular, it can pass through connectors which are themselves mounted on threaded protrusions carried by plates, bone anchoring screws or hooks which are placed opposite the pedicles or on the posterior arches or transverse processes of the vertebrae.

For the osteosynthesis devices fitted by a posterior approach, anchoring the rod on the vertebrae by means of hooks placed on a vertebra is particularly indicated. To this end, anchoring means have been conceived in which a hook connected to the rod of the device by a connector, as indicated above, is connected to a second hook by means of a rod of small diameter. The second hook is placed at a distance from the first one on the same vertebra or, if appropriate, on another vertebra. Such means are described in particular in document FR-A-2 816 196. In said document, the rod of small diameter is threaded and is inserted into an internally threaded hole in the head of the hook which bears the connector. In document FR-A-2 767 263, the hook on which the rod of the osteosynthesis device is fixed comprises, on its head, two channels: one in which the rod of the osteosynthesis device is installed (therefore no connector is used here), the other in which the rod of small diameter is installed, which connects the hook to the other hook. The rods are immobilized in the head of the hook by threaded plugs.

These means of fixation have the disadvantage of having relatively complex structures and of requiring, for their installation, a number of manoeuvres and a length of time which it would be desirable to reduce.

The object of the invention is to make available novel means of fixing the rod of a spinal osteosyntheis device on a vertebra, which afford such reductions and are thus more convenient and economic to use than the existing means.

To this end, the subject of the invention is a device for connecting the rod of a spinal osteosynthesis device to a vertebra or to two vertebrae, of the type comprising a first hook provided with a threaded protrusion for fitting a connector in which said rod is inserted, a second hook, a rod of small diameter connecting the two hooks, and means for immobilizing the ends of said rod of small diameter on the heads of the hooks, characterized in that the end of the rod of small diameter in contact with said first hook comprises an oblong aperture, and in that the means for immobilizing this end on the head of the first hook are formed by a nut intended to cooperate with the connector.

Said nut can be a divisible nut, and it is its lower part that is intended to cooperate with the connector.

Said protrusion can have an angulation with respect to the upper surface of the first hook.

Said end of the rod of small diameter having the oblong aperture is flattened in such a way as to cooperate with a plane upper surface of the head of the first hook.

The head of the second hook can comprise a groove for insertion of an end of the rod of small diameter, and the means for immobilizing said end in said head are formed by a threaded plug.

The invention also relates to a spinal osteosynthesis device intended to be fitted by a posterior approach, of the type comprising a rod, connectors, and means for joining said connectors to the patient's vertebrae, characterized in that the means for joining at least one of said connectors to a vertebra or to two vertebrae are formed by a device of the above type.

As will have been appreciated, the invention consists principally in conferring a particular configuration on the rod of small diameter connecting the two hooks. The end of the rod to be fixed to the hook supporting the protrusion is provided with an oblong aperture through which the protrusion is intended to pass. This particular feature in particular makes it very easy for the surgeon to regulate the distance between the two hooks.

The invention will be better understood on reading the following description in which reference is made to the following attached figures, where:
- Figure 1 shows the main elements of the device according to the invention in a perspective view;
- Figures 2 to 5 show the different stages involved in fitting a spinal osteosynthesis device using the invention.

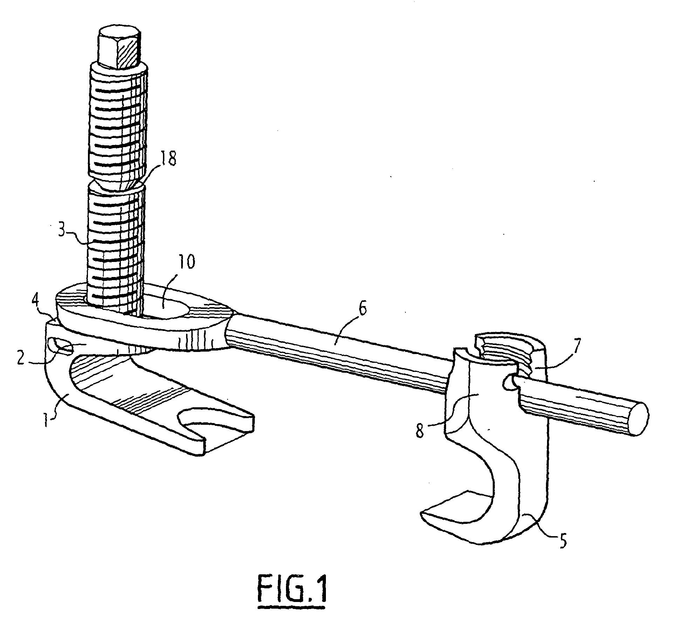

As will be seen from Figure 1, the device according to the invention comprises a first hook 1 intended to be attached to a lamina or opposite a pedicle of a vertebra. Its head 2 supports a threaded protrusion 3 which is substantially perpendicular to the flat upper surface 4 of the head 2. The device comprises a second hook 5 intended to be attached to the posterior arch or to the transverse process of said vertebra (or, if appropriate, of a different vertebra). As is known, these two hooks 1, 5 are connected via a rod 6 of small diameter. The latter has one of its ends simply placed in a groove in the head 8 of the second hook 5, where it is free in translation and in rotation before being immobilized there by a threaded plug 9. According to the invention, the other end of the rod 6 is flattened and has an oblong aperture 10 with a length of the order of 10 mm, for example. The threaded protrusion 3 can be fitted through said aperture 10 in such a way that the flattened end of the rod 6 rests on the head 4 of the first hook 1 and can be immobilized against it by a nut, an example of which will be described further below. The rod 6 has a diameter generally of the order of 3 mm; it is referred to as being "of small diameter" in this description simply by contrast to the rod 13 of the osteosynthesis device which generally has a substantially greater diameter (of the order of 5.5 mm, for example).

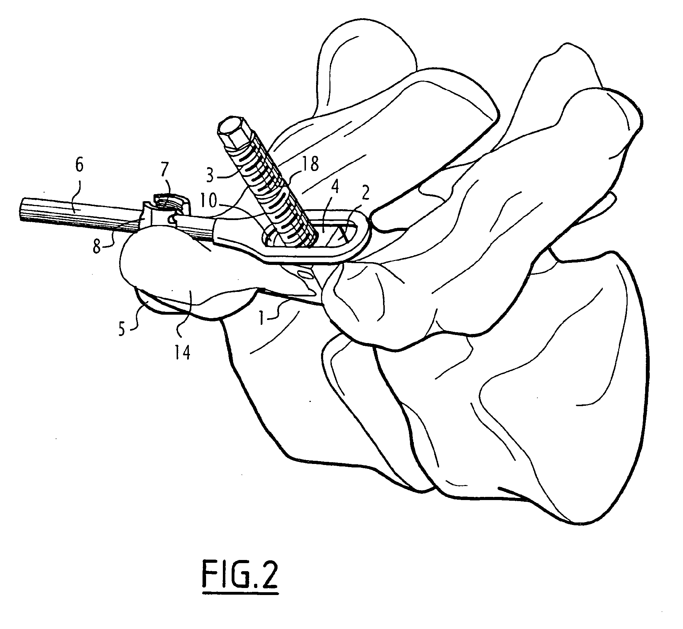

Figure 2 shows the first stage involved in fitting the device according to the invention on a vertebra 14 of the patient. The first hook 1 is installed opposite a pedicle of the vertebra 14, and the second hook 5 on the transverse process of the same vertebra 14, it being understood that this arrangement is just one example and that any other type of location of the hooks on the vertebra could be conceivable. The rod 6 of small diameter connecting the two hooks 1, 5 is arranged as in Figure 1. It will be noted that the oblong shape of the aperture 10 allows the surgeon great latitude in positioning the rod 6 in the best possible location without difficulty. Likewise, the angular position of the second hook 5 is established without difficulty.

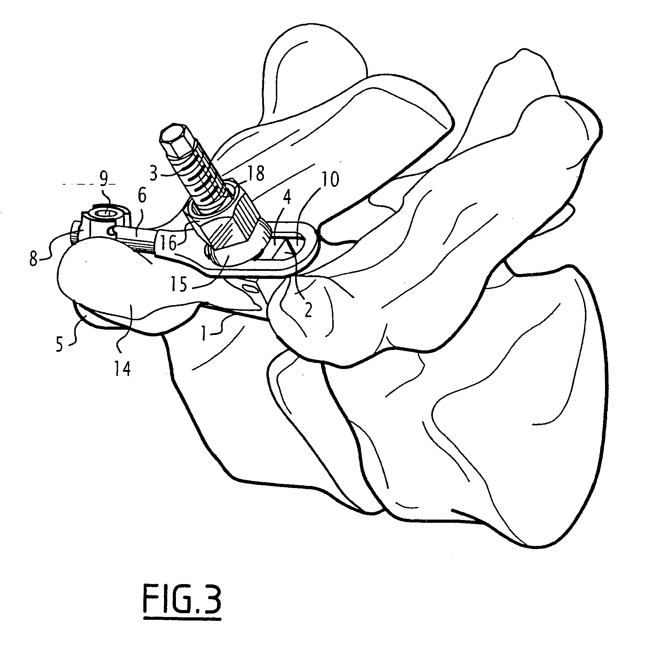

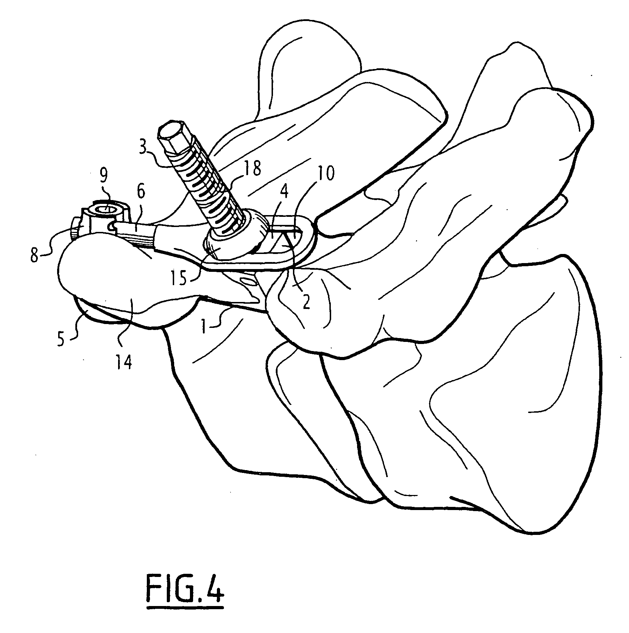

Figure 3 shows the device after the second stage involved in fitting it. The rod 6 of small diameter has been immobilized on the hooks 1, 5, on the one hand by the threaded plug 9 screwed into the groove in the head 8 of the second hook 5, and, on the other hand, by a nut fitted on the protrusion 3. This nut comprises two parts separated by a line of lesser strength (not visible in Figure 4) which makes the nut divisible. The lower part 15 is designed with a conical (or spherical) upper surface intended to cooperate with the connector 11. The upper part 16 has flat faces allowing the nut to be tightened by means of a suitable tool. Before or after the rod 6 is immobilized, it is preferable to cut off its part extending beyond the second hook 5.

Figure 4 shows the device after the third stage involved in fitting it, which stage has consisted in removing the upper part 16 of the nut fitted on the protrusion 3. All that remains of this nut, therefore, is its lower part 15 which is ready to receive the connector 11.

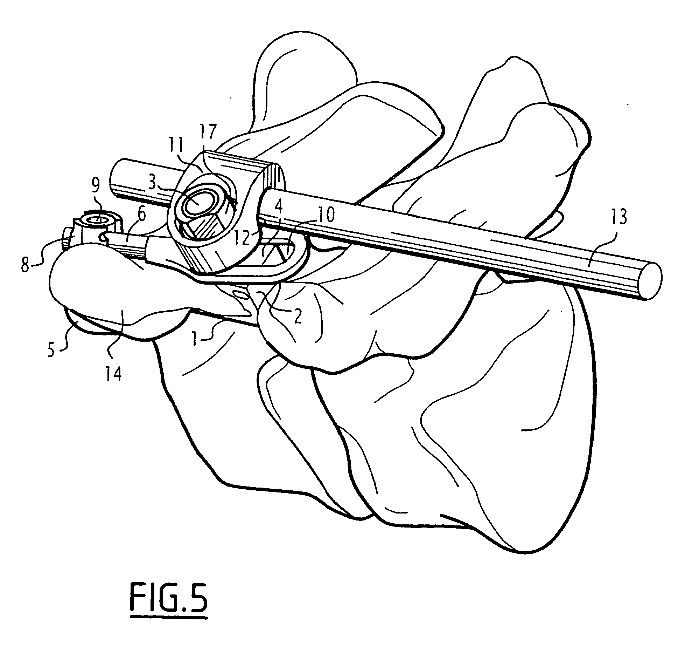

Figure 5 shows the device according to the invention in its final state, after the rod 13 of the spinal osteosynthesis device has been connected to it. To do this, the connector 11, in whose longitudinal orifice 12 the rod 13 has first been fitted, is placed on the protrusion 3 and then clamped by a nut 17 which has the dual function of clamping the connector 11 on the remaining part 15 of the nut and of wedging the rod 13 inside the connector 11. After the nut 17 has been tightened, the part of the protrusion 3 emerging from it is, as is customary, cut off by the surgeon along a line of lesser resistance 18.

The example which has just been described and shown is of course non-limiting, and various modifications can be made to it.

For example, the device can be installed not on just one vertebra, but on two different vertebrae, in such a way as to form a sub-assembly of vertebrae to which the rod 13 is connected. The designs of the hooks 1, 5 can be modified depending on the site of installation envisaged.

The rod 6 of small diameter can be connected to the second hook 5 by means other than those shown.

The configuration of the connector 11 can be different, in which case, consequently, the configuration of the nut 15, 16 clamping the rod 6 of small diameter on the head 2 of the first hook 1 must be modified. It is not necessary for this nut 15, 16 to be divisible.

In the example shown, the end of the rod 6 with the oblong aperture 10 is flattened in order to cooperate with the plane upper surface 4 of the first hook 1, but other designs are conceivable, for example a sphere-to-sphere contact between the rod 6 and the hook 1.

In the example shown, the protrusion 3 is made in one piece with the first hook 1 and perpendicular to the latter's upper surface 4, but it can also be formed by a separate component connected to the first hook 1 in a way which allows the protrusion 3 to be angled with respect to the surface 4 of the hook 1 inside a cone. This angulation, which is normally of the order of at most 30°, can make it easier to fit the osteosynthesis device because it permits only approximate preliminary bending of the rod 13 prior to insertion of the connector 11. If the configurations of the means for connecting the protrusion 3 to the hook 1, and of the nut for immobilizing the rod 6 of small diameter on the hook 1, so permit, this angulation can, if appropriate, persist after the device has been immobilized. Means for ensuring this angulation are well known in the prior art, for example in document WO-A-02/38061, where they are applied to plates.

The device for spinal osteosynthesis by a posterior approach, with which the connection device according to the invention is made integral, can, depending on the surgeon's preference and on its location on the spine, comprise only connection devices according to the invention, or simultaneously comprise one or more connection devices according to the invention and one or more connection devices of other types.

## Claims

1. Device for connecting the rod (13) of a spinal osteosynthesis device to a vertebra (14) or to two vertebrae, of the type comprising a first hook (1) provided with a threaded protrusion (3) for fitting a connector (11) in which said rod is inserted, a second hook (5), a rod (6) of small diameter connecting the two hooks (1, 5), and means for immobilizing the ends of said rod (6) of small diameter on the heads (2, 8) of the hooks (1, 5), **characterized in that** the end of the rod (6) of small diameter in contact with said first hook comprises an oblong aperture (10), and **in that** the means for immobilizing this end on the head (2) of the first hook (1) are formed by a nut (15, 16) intended to cooperate with the connector (11).

2. Device according to Claim 1, **characterized in that** said nut (15, 16) is a divisible nut, and **in that** it is its lower part (15) that is intended to cooperate with the connector (11).

3. Device according to Claim 1 or 2, **characterized in that** said protrusion (3) can have an angulation with respect to the upper surface (4) of the first hook (1).

4. Device according to one of Claims 1 to 3, **characterized in that** said end of the rod (6) of small diameter having the oblong aperture (10) is flattened in such a way as to cooperate with a plane upper surface (4) of the head (2) of the first hook (1).

5. Device according to one of Claims 1 to 4, **characterized in that** the head (8) of the second hook (5) comprises a groove (7) for insertion of an end of the rod (6) of small diameter, and **in that** the means for immobilizing said end in said head (8) are formed by a threaded plug (9).

6. Spinal osteosynthesis device intended to be fitted by a posterior approach, of the type comprising a rod (13), connectors (11), and means for joining said connectors (11) to the patient's vertebrae, **characterized in that** the means for joining at least one of said connectors (11) to a vertebra (14) or to two vertebrae are formed by a device according to one of Claims 1 to 5.

## Patentansprüche

1. Vorrichtung zum Verbinden des Stabs (13) einer spinalen Osteosynthesevorrichtung an einem Wirbel (14) oder an zwei Wirbeln, der Gattung mit einem ersten Haken (1), der mit einem mit Gewinde versehenen Vorsprung (3) zum Anbringen eines Verbinders (11) ausgestattet ist, in dem der Stab eingesetzt ist, einem zweiten Haken (5), einem Stab (6) kleinen Durchmessers, der die beiden Haken (1, 5) verbindet, und Mitteln zur bewegungslosen Festlegung der Enden des Stabs (6) kleinen Durchmessers an den Köpfen (2, 8) der Haken (1, 5), **dadurch gekennzeichnet, dass** das Ende des Stabs (6) kleinen Durchmessers, das mit dem ersten Haken in Berührung steht, eine längliche Öffnung (10) aufweist und das die Mittel zur bewegungslosen Festlegung dieses Endes an dem Kopf (2) des ersten Hakens (1) durch eine Mutter (15, 16) gebildet sind, die zur Zusammenarbeit mit dem Verbinder (11) bestimmt ist.

2. Vorrichtung nach Anspruch 1, **dadurch gekennzeichnet, dass** die Mutter (15, 16) eine teilbare Mutter ist und dass es ihr unterer Teil (15) ist, der zur Zusammenarbeit mit dem Verbinder (11) bestimmt ist.

3. Vorrichtung nach Anspruch 1 oder 2, **dadurch gekennzeichnet, dass** der Vorsprung (3) eine Winkligkeit bzw. Neigung gegenüber der oberen Fläche (4) des ersten Hakens (1) aufweisen kann.

4. Vorrichtung nach einem der Ansprüche 1 bis 3, **dadurch gekennzeichnet, dass** das Ende des Stabs (6) kleinen Durchmessers, das die längliche Öffnung (10) aufweist, in einer solchen Weise abgeflacht ist, dass es mit einer planen oberen Fläche (4) des Kopfes (2) des ersten Hakens (1) zusammenarbeitet.

5. Vorrichtung nach einem der Ansprüche 1 bis 4, **dadurch gekennzeichnet, dass** der Kopf (8) des zweiten Hakens (5) eine Nut (7) zum Einsetzen eines Endes des Stabs (6) kleinen Durchmessers aufweist und dass die Mittel zur bewegungslosen Festlegung des Endes in dem Kopf (8) durch einen mit Gewinde versehenen Stopfen (9) gebildet sind.

6. Spinale Osteosynthesevorrichtung, die zur Anbringung von hinten bestimmt ist, der Gattung mit einem Stab (13), Verbindern (11) und Mitteln zum Verbinden der Verbinder (11) mit den Wirbeln eines Patienten, **dadurch gekennzeichnet, dass** die Mittel zum Verbinden mindestens eines der Verbinder (11) an einem Wirbel (14) oder an zwei Wirbeln durch eine Vorrichtung nach einem der Ansprüche 1 bis 5 gebildet sind.

## Revendications

1. Dispositif de connexion de la tige (13) d'un dispositif d'ostéosynthèse rachidienne à une vertèbre (14) ou à deux vertèbres, du type comprenant un premier crochet (1) muni d'une saillie filetée (3) destinée à fixer un dispositif de connexion (11) dans lequel ladite tige est insérée, un second crochet (5), une tige (6) de petit diamètre qui relie les deux crochets (1, 5), et des moyens destinés à immobiliser les extrémités de ladite tige (6) de petit diamètre sur les têtes (2, 8) des crochets (1, 5), **caractérisé en ce que** l'extrémité de la tige (6) de petit diamètre en contact avec ledit premier crochet comprend une ouverture oblongue (10), et **en ce que** les moyens destinés à immobiliser cette extrémité sur la tête (2) du premier crochet (1) sont formés par un écrou (15, 16) destiné à coopérer avec le dispositif de connexion (11).

2. Dispositif selon la revendication 1, **caractérisé en ce que** ledit écrou (15, 16) est un écrou divisible, et **en ce que** c'est sa partie inférieure (15) qui est destinée à coopérer avec le dispositif de connexion (11).

3. Dispositif selon la revendication 1 ou 2, **caractérisé en ce que** ladite saillie (3) peut présenter une inclinaison par rapport à la surface supérieure (4) du premier crochet (1).

4. Dispositif selon l'une quelconque des revendications 1 à 3, **caractérisé en ce que** ladite extrémité de la tige (6) de petit diamètre qui comporte l'ouverture oblongue (10) est aplatie de manière à coopérer avec une surface supérieure plane (4) de la tête (2) du premier crochet (1).

5. Dispositif selon l'une quelconque des revendications 1 à 4, **caractérisé en ce que** la tête (8) du second crochet (5) comprend une rainure (7) pour l'insertion d'une extrémité de la tige (6) de petit diamètre, et **en ce que** les moyens destinés à immobiliser ladite extrémité dans ladite tête (8) sont formés par un bouchon fileté (9).

6. Dispositif d'ostéosynthèse rachidienne destiné à être fixé par une approche postérieure, du type comprenant une tige (13), des dispositifs de connexion (11), ainsi que des moyens destinés à joindre lesdits dispositifs de connexion (11) aux vertèbres du patient, **caractérisé en ce que** les moyens destinés à joindre au moins l'un des dispositifs de connexion (11) à une ou deux vertèbres (14) sont formés par un dispositif selon l'une des revendications 1 à 5.
